# EUROPEAN PATENT APPLICATION

(11) **EP 1 217 069 A2**
(43) Date of publication of application: **26.06.2002**
(21) Application number: 01128596.2
(22) Date of filing: 30.11.2001
(51) Int. Cl.: C12N 15/54, C12N 9/10, C12P 13/06, C12P 13/08, C12P 13/22, C12Q 1/68

(54) **Nucleotide sequences which code for the ilvE gene**

(30) Priority: 20.12.2000 DE 10063314
(71) Applicant: Degussa AG, 40474 Düsseldorf (DE)
(72) Inventor: Bathe, Brigitte, Dr., 33154 Salzkotten (DE); Bastuck, Christine, 33602 Bielefeld (DE); Tauch, Andreas, Dr., 33647 Bielefeld (DE); McHardy, Alice, 33619 Bielefeld (DE)

(57) **Abstract**

The invention relates to an isolated polynucleotide comprising a polynucleotide sequence chosen from the group consisting of
a) polynucleotide which is identical to the extent of at least 70% to a polynucleotide which codes for a polypeptide which comprises the amino acid sequence of SEQ ID No. 2,
b) polynucleotide which codes for a polypeptide which comprises an amino acid sequence which is identical to the extent of at least 70% to the amino acid sequence of SEQ ID No. 2,
c) polynucleotide which is complementary to the polynucleotides of a) or b), and
d) polynucleotide comprising at least 15 successive nucleotides of the polynucleotide sequence of a), b) or c),
and a process for the fermentative preparation of L-amino acids using coryneform bacteria in which at least the ilvE gene is present in enhanced form, and the use of polynucleotides which comprise the sequences according to the invention as hybridization probes.

## Description

The invention provides nucleotide sequences from coryneform bacteria which code for the ilvE gene and a process for the fermentative preparation of amino acids using bacteria in which the endogenous ilvE gene is enhanced.

### Prior art

L-Amino acids, in particular L-leucine, L-valine, L-isoleucine and L-phenylalanine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and produce amino acids are obtained in this manner.

Methods of the recombinant DNA technique have also been employed for some years for improving the strain of Corynebacterium strains which produce L-amino acid, by amplifying individual amino acid biosynthesis genes and investigating the effect on the amino acid production.

### Object of the invention

The inventors had the object of providing new measures for improved fermentative preparation of amino acids.

### Description of the invention

Where L-amino acids or amino acids are mentioned in the following, this means one or more amino acids, including their salts, chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-leucine, L-valine, L-isoleucine and L-phenylalanine are particularly preferred.

The invention provides an isolated polynucleotide from coryneform bacteria, comprising a polynucleotide sequence which codes for the ilvE gene, chosen from the group consisting of
a) polynucleotide which is identical to the extent of at least 70% to a polynucleotide which codes for a polypeptide which comprises the amino acid sequence of SEQ ID No. 2,
b) polynucleotide which codes for a polypeptide which comprises an amino acid sequence which is identical to the extent of at least 70% to the amino acid sequence of SEQ ID No. 2,
c) polynucleotide which is complementary to the polynucleotides of a) or b), and
d) polynucleotide comprising at least 15 successive nucleotides of the polynucleotide sequence of a), b) or c), the polypeptide preferably having the activity of transaminase E.

The invention also provides the abovementioned polynucleotide, this preferably being a DNA which is capable of replication, comprising:
- (i): the nucleotide sequence shown in SEQ ID No. 1, or
- (ii): at least one sequence which corresponds to sequence (i) within the range of the degeneration of the genetic code, or
- (iii): at least one sequence which hybridizes with the sequence complementary to sequence (i) or (ii), and optionally
- (iv): sense mutations of neutral function in (i) which do not modify the activity of the protein/polypeptide.

Finally, the invention also provides polynucleotides chosen from the group consisting of
a) polynucleotides comprising at least 15 successive nucleotides chosen from the nucleotide sequence of SEQ ID No. 1 between positions 1 and 220,
b) polynucleotides comprising at least 15 successive nucleotides chosen from the nucleotide sequence of SEQ ID No. 1 between positions 221 and 1324,
c) polynucleotides comprising at least 15 successive nucleotides chosen from the nucleotide sequence of SEQ ID No. 1 between positions 1325 and 1453.

The invention also provides
a polynucleotide, in particular DNA, which is capable of replication and comprises the nucleotide sequence as shown in SEQ ID No. 1;
a polynucleotide which codes for a polypeptide which comprises the amino acid sequence as shown in SEQ ID No. 2;
a vector containing the polynucleotide according to the invention, in particular a shuttle vector or plasmid vector, and
coryneform bacteria which contain the vector or in which the endogenous ilvE gene is enhanced.

The invention also provides polynucleotides, which substantially comprise a polynucleotide sequence, which are obtainable by screening by means of hybridization of a corresponding gene library of a coryneform bacterium, which comprises the complete gene or parts thereof, with a probe which comprises the sequence of the polynucleotide according to the invention according to SEQ ID No.1 or a fragment thereof, and isolation of the polynucleotide sequence mentioned.

Polynucleotides which comprise the sequences according to the invention are suitable as hybridization probes for RNA, cDNA and DNA, in order to isolate, in the full length, nucleic acids or polynucleotides or genes which code for transaminase E or to isolate those nucleic acids or polynucleotides or genes which have a high similarity of sequence with the sequence of the ilvE gene. They are also suitable for incorporation into so-called "arrays", "micro arrays" or "DNA chips" in order to detect and determine the corresponding polynucleotides

Polynucleotides which comprise the sequences according to the invention are furthermore suitable as primers with the aid of which DNA of genes which code for transaminase E can be prepared by the polymerase chain reaction (PCR).

Such oligonucleotides which serve as probes or primers comprise at least 25, 26, 27, 28, 29 or 30, preferably at least 20, 21, 22, 23 or 24, very particularly preferably at least 15, 16, 17, 18 or 19 successive nucleotides. Oligonucleotides with a length of at least 31, 32, 33, 34, 35, 36, 37, 38, 39 or 40, or at least 41, 42, 43, 44, 45, 46, 47, 48, 49 or 50 nucleotides are also suitable. Oligonucleotides with a length of at least 100, 150, 200, 250 or 300 nucleotides are optionally also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

The polynucleotides according to the invention include a polynucleotide according to SEQ ID No. 1 or a fragment prepared therefrom and also those which are at least in particular 70% to 80%, preferably at least 81% to 85%, particularly preferably at least 86% to 90%, and very particularly preferably at least 91%, 93%, 95%, 97% or 99% identical to the polynucleotide according to SEQ ID No. 1 or a fragment prepared therefrom.

"Polypeptides" are understood as meaning peptides or proteins which comprise two or more amino acids bonded via peptide bonds.

The polypeptides according to the invention include a polypeptide according to SEQ ID No. 2, in particular those with the biological activity of transaminase E, and also those which are at least 70% to 80%, preferably at least 81% to 85%, particularly preferably at least 86% to 90%, and very particularly preferably at least 91%, 93%, 95%, 97% or 99% identical to the polypeptide according to SEQ ID No. 2 and have the activity mentioned.

The invention furthermore relates to a process for the fermentative preparation of amino acids chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine using coryneform bacteria which in particular already produce amino acids and in which the nucleotide sequences which code for the ilvE gene are enhanced, in particular over-expressed.

The term "enhancement" in this connection describes the increase in the intracellular activity of one or more enzymes in a microorganism which are coded by the corresponding DNA, for example by increasing the number of copies of the gene or genes, using a potent promoter or using a gene which codes for a corresponding enzyme having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The microorganisms which the present invention provides can produce L-amino acids from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 and
Brevibacterium divaricatum ATCC14020
and L-amino acid-producing mutants or strains prepared therefrom.

The new ilvE gene from C. glutamicum which codes for the enzyme transaminase E (EC 2.6.1.42) has been isolated.

To isolate the ilvE gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einführung in die Gentechnologie [Genes and Clones, An Introduction to Genetic Engineering] (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495 -508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575).

Börmann et al. (Molecular Microbiology 6(3), 317-326) (1992)) in turn describe a gene library of C. glutamicum ATCC13032 using the cosmid pHC79 (Hohn and Collins, Gene 11, 291-298 (1980)).

To prepare a gene library of C. glutamicum in E. coli it is also possible to use plasmids such as pBR322 (Bolivar, Life Sciences, 25, 807-818 (1979)) or pUC9 (Vieira et al., 1982, Gene, 19:259-268). Suitable hosts are, in particular, those E. coli strains which are restriction- and recombination-defective. An example of these is the strain DH5αmcr, which has been described by Grant et al. (Proceedings of the National Academy of Sciences USA, 87 (1990) 4645-4649). The long DNA fragments cloned with the aid of cosmids can in turn be subcloned in the usual vectors suitable for sequencing and then sequenced, as is described e.g. by Sanger et al. (Proceedings of the National Academy of Sciences of the United States of America, 74:5463-5467, 1977).

The resulting DNA sequences can then be investigated with known algorithms or sequence analysis programs, such as e.g. that of Staden (Nucleic Acids Research 14, 217-232(1986)), that of Marck (Nucleic Acids Research 16, 1829-1836 (1988)) or the GCG program of Butler (Methods of Biochemical Analysis 39, 74-97 (1998)).

The new DNA sequence of C. glutamicum which codes for the ilvE gene and which, as SEQ ID No. 1, is a constituent of the present invention has been found. The amino acid sequence of the corresponding protein has furthermore been derived from the present DNA sequence by the methods described above. The resulting amino acid sequence of the ilvE gene product is shown in SEQ ID No. 2. It is known that enzymes endogenous in the host can split off the N-terminal amino acid methionine or formylmethionine of the protein formed.

Coding DNA sequences which result from SEQ ID No. 1 by the degeneracy of the genetic code are also a constituent of the invention. In the same way, DNA sequences which hybridize with SEQ ID No. 1 or parts of SEQ ID No. 1 are a constituent of the invention. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. Such mutations are also called, inter alia, neutral substitutions. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the invention.

In the same way, DNA sequences which hybridize with SEQ ID No. 1 or parts of SEQ ID No. 1 are a constituent of the invention. Finally, DNA sequences which are prepared by the polymerase chain reaction (PCR) using primers which result from SEQ ID No. 1 are a constituent of the invention. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology (1991) 41: 255-260). The hybridization takes place under stringent conditions, that is to say only hybrids in which the probe and target sequence, i. e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50ºC - 68ºC, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50ºC - 68ºC being established. It is optionally possible to lower the salt concentration to 0.1x SSC. Polynucleotide fragments which are, for example, at least 70% or at least 80% or at least 90% to 95% identical to the sequence of the probe employed can be isolated by increasing the hybridization temperature stepwise from 50ºC to 68ºC in steps of approx. 1 - 2ºC. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that coryneform bacteria produce amino acids in an improved manner after over-expression of the ilvE gene.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative amino acid production. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification 0 472 869, in US Patent 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15 - 24 (1993)), in Japanese Laid-Open Specification JP-A-10-229891, in Jensen and Hammer (Biotechnology and Bioengineering 58, 191-195 (1998)), in Makrides (Microbiological Reviews 60:512-538 (1996)) and in known textbooks of genetics and molecular biology.

By way of example, for enhancement the ilvE gene according to the invention was over-expressed with the aid of episomal plasmids. Suitable plasmids are those which are replicated in coryneform bacteria. Numerous known plasmid vectors, such as e.g. pZ1 (Menkel et al., Applied and Environmental Microbiology (1989) 64: 549-554), pEKEx1 (Eikmanns et al., Gene 102:93-98 (1991)) or pHS2-1 (Sonnen et al., Gene 107:69-74 (1991)) are based on the cryptic plasmids pHM1519, pBL1 or pGA1. Other plasmid vectors, such as e.g. those based on pCG4 (US-A 4,489,160), or pNG2 (Serwold-Davis et al., FEMS Microbiology Letters 66, 119-124 (1990)), or pAG1 (US-A 5,158,891), can be used in the same manner.

Plasmid vectors which are furthermore suitable are also those with the aid of which the process of gene amplification by integration into the chromosome can be used, as has been described, for example, by Reinscheid et al. (Applied and Environmental Microbiology 60, 126-132 (1994)) for duplication or amplification of the hom-thrB operon. In this method, the complete gene is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pGEM-T (Promega Corporation, Madison, WI, USA), pCR2.1-TOPO (Shuman (1994). Journal of Biological Chemistry 269:32678-84; US-A 5,487,993), pCR®Blunt (Invitrogen, Groningen, Holland; Bernard et al., Journal of Molecular Biology, 234: 534-541 (1993)), pEM1 (Schrumpf et al, 1991, Journal of Bacteriology 173:4510-4516) or pBGS8 (Spratt et al.,1986, Gene 41: 337-342). The plasmid vector which contains the gene to be amplified is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross over" event, the resulting strain contains at least two copies of the gene in question.

In addition, it may be advantageous for the production of L-amino acids to enhance, in particular over-express one or more enzymes of the particular biosynthesis pathway, of glycolysis, of anaplerosis, of the citric acid cycle, of the pentose phosphate cycle, of amino acid export and optionally regulatory proteins, in addition to the ilvE gene.

Thus, for the preparation of L-amino acids, in addition to enhancement of the ilvE gene, one or more endogenous genes chosen from the group consisting of
- the gap gene which codes for glyceraldehyde 3-phosphate dehydrogenase (Eikmanns (1992), Journal of Bacteriology 174:6076-6086),
- the tpi gene which codes for triose phosphate isomerase (Eikmanns (1992), Journal of Bacteriology 174:6076-6086),
- the pgk gene which codes for 3-phosphoglycerate kinase (Eikmanns (1992), Journal of Bacteriology 174:6076-6086),
- the zwf gene which codes for glucose 6-phosphate dehydrogenase (JP-A-09224661),
- the mqo gene which codes for malate-quinone oxidoreductase (Molenaar et al., European Journal of Biochemistry 254, 395-403 (1998)),
- the lysC gene which codes for a feed-back resistant aspartate kinase (Accession No.P26512),
- the hom gene which codes for homoserine dehydrogenase (EP-A 0131171),
- the ilvA gene which codes for threonine dehydratase (Möckel et al., Journal of Bacteriology (1992) 8065-8072)) or the ilvA(Fbr) allele which codes for a "feed back resistant" threonine dehydratase (Möckel et al., (1994) Molecular Microbiology 13: 833-842),
- the ilvBN gene which codes for acetohydroxy-acid synthase (EP-B 0356739),
- the ilvD gene which codes for dihydroxy-acid dehydratase (Sahm and Eggeling (1999) Applied and Environmental Microbiology 65: 1973-1979),
- the leuB gene which codes for 3-isopropyl malate dehydrogenase (Patek et al. (1998) Applied and Microbiology Biotechnologie [sic] 50: 42-47)
- the brnE gene which codes for valine export (DE: 19951708.8)
- the leuA gene which codes for isopropyl malate synthase(Patek et al.(1994) Applied and Environmental Microbiology 60 (1): 133-140)
- the pyc gene which codes for pyruvate carboxylase (Peters-Wendisch et al.(Microbiology 144, 915 - 927 (1998))
- the zwa1 gene which codes for the Zwa1 protein (DE: 19959328.0, DSM 13115),
can be enhanced, in particular over-expressed.

It may furthermore be advantageous for the production of L-amino acids, in addition to the enhancement of the ilvE gene, for one or more of the genes chosen from the group consisting of:
- the pck gene which codes for phosphoenol pyruvate carboxykinase (DE 199 50 409.1; DSM 13047),
- the dapA gene which codes for dihydrodipicolinate synthase (EP-B 0 197 335),
- the pgi gene which codes for glucose 6-phosphate isomerase (US 09/396,478; DSM 12969),
- the poxB gene which codes for pyruvate oxidase (DE: 1995 1975.7; DSM 13114),
- the zwa2 gene which codes for the Zwa2 protein (DE: 19959327.2, DSM 13113)
to be attenuated, in particular for the expression thereof to be reduced.

In addition to over-expression of the ilvE gene it may furthermore be advantageous for the production of amino acids to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of amino acids. A summary of known culture methods is described in the textbook by Chmiel (Bioprozesstechnik 1. Einführung in die Bioverfahrenstechnik [Bioprocess Technology 1. Introduction to Bioprocess Technology] (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20ºC to 45ºC, and preferably 25ºC to 40ºC. Culturing is continued until a maximum of the desired product has formed. This target is usually reached within 10 hours to 160 hours.

Methods for the determination of L-amino acids are known from the prior art. The analysis can thus be carried out, for example, as described by Spackman et al. (Analytical Chemistry, 30, (1958), 1190) by ion exchange chromatography with subsequent ninhydrin derivatization, or it can be carried out by reversed phase HPLC, for example as described by Lindroth et al. (Analytical Chemistry (1979) 51: 1167-1174).

The process according to the invention is used for fermentative preparation of amino acids.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual (1989) Cold Spring Harbor Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from Corynebacterium glutamicum ATCC 13032

Chromosomal DNA from Corynebacterium glutamicum ATCC 13032 is isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments are dephosphorylated with shrimp alkaline phosphatase (Roche Diagnostics GmbH, Mannheim, Germany, Product Description SAP, Code no. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vector Kit, Code no. 251301) is cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA is then cleaved with the restriction enzyme BamHI (Amersham Pharmacia, Freiburg, Germany, Product Description BamHI, Code no. 27-0868-04). The cosmid DNA treated in this manner is mixed with the treated ATCC13032 DNA and the batch is treated with T4 DNA ligase (Amersham Pharmacia, Freiburg, Germany, Product Description T4-DNA-Ligase, Code no.27-0870-04). The ligation mixture is then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Research 16:1563-1575) the cells are taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology, 1:190) with 100 mg/l ampicillin. After incubation overnight at 37ºC, recombinant individual clones are selected.

### Example 2

### Isolation and sequencing of the ilvE gene

The cosmid DNA of an individual colony is isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments are dephosphorylated with shrimp alkaline phosphatase (Roche Diagnostics GmbH, Mannheim, Germany, Product Description SAP, Product No. 1758250). After separation by gel electrophoresis, the cosmid fragments in the size range of 1500 to 2000 bp are isolated with the QiaExII Gel Extraction Kit (Product No. 20021, Qiagen, Hilden, Germany).

The DNA of the sequencing vector pZero-1, obtained from Invitrogen (Groningen, Holland, Product Description Zero Background Cloning Kit, Product No. K2500-01), is cleaved with the restriction enzyme BamHI (Amersham Pharmacia, Freiburg, Germany, Product Description BamHI, Product No. 27-0868-04). The ligation of the cosmid fragments in the sequencing vector pZero-1 is carried out as described by Sambrook et al. (1989, Molecular Cloning: A laboratory Manual, Cold Spring Harbor), the DNA mixture being incubated overnight with T4 ligase (Pharmacia Biotech, Freiburg, Germany). This ligation mixture is then electroporated (Tauch et al. 1994, FEMS Microbiol Letters, 123:343-7) into the E. coli strain DH5αMCR (Grant, 1990, Proceedings of the National Academy of Sciences U.S.A., 87:4645-4649) and plated out on LB agar (Lennox, 1955, Virology, 1:190) with 50 mg/l zeocin.

The plasmid preparation of the recombinant clones is carried out with a Biorobot 9600 (Product No. 900200, Qiagen, Hilden, Germany). The sequencing is carried out by the dideoxy chain termination method of Sanger et al. (1977, Proceedings of the National Academy of Sciences U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction are carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained are then processed using the Staden program package (1986, Nucleic Acids Research, 14:217-231) version 97-0. The individual sequences of the pzero1 derivatives are assembled to a continuous contig.

The computer-assisted coding region analysis is prepared with the XNIP program (Staden, 1986, Nucleic Acids Research 14:217-231).

The resulting nucleotide sequence is shown in SEQ ID No. 1. Analysis of the nucleotide sequence shows an open reading frame of 1103 base pairs, which is called the ilvE gene. The ilvE gene codes for a protein of 367 amino acids.

## Claims

1. Isolated polynucleotide from coryneform bacteria, comprising a polynucleotide sequence which codes for the ilvE gene, chosen from the group consisting of
a) polynucleotide which is identical to the extent of at least 70% to a polynucleotide which codes for a polypeptide which comprises the amino acid sequence of SEQ ID No. 2,
b) polynucleotide which codes for a polypeptide which comprises an amino acid sequence which is identical to the extent of at least 70% to the amino acid sequence of SEQ ID No. 2,
c) polynucleotide which is complementary to the polynucleotides of a) or b), and
d) polynucleotide comprising at least 15 successive nucleotides of the polynucleotide sequence of a), b) or c)
the polypeptide preferably having the activity of transaminase E.

2. Polynucleotide according to claim 1, wherein the polynucleotide is a preferably recombinant DNA which is capable of replication in coryneform bacteria.

3. Polynucleotide according to claim 1, wherein the polynucleotide is an RNA.

4. Polynucleotide according to claim 2, comprising the nucleic acid sequence as shown in SEQ ID No. 1.

5. DNA according to claim 2 which is capable of replication, comprising
(i) the nucleotide sequence shown in SEQ ID No. 1, or
(ii) at least one sequence which corresponds to sequence (i) within the range of the degeneration of the genetic code, or
(iii) at least one sequence which hybridizes with the sequence complementary to sequence (i) or (ii), and optionally
(iv) sense mutations of neutral function in (i).

6. DNA according to claim 5 which is capable of replication, **characterized in that** the hybridization is carried out under a stringency corresponding to at most 2x SSC.

7. Polynucleotide sequence according to claim 1, which codes for a polypeptide which comprises the amino acid sequence shown in SEQ ID No. 2.

8. Coryneform bacteria in which the ilvE gene is enhanced, in particular over-expressed.

9. Process for the fermentative preparation of L-amino acids, in particular L-leucine, L-valine, L-isoleucine and L-phenylalanine, **characterized in that** the following steps are carried out:
a) fermentation of the coryneform bacteria which produce the desired L-amino acid and in which at least the endogenous ilvE gene or nucleotide sequences which code for it are enhanced, in particular over-expressed;
b) concentration of the L-amino acid in the medium or in the cells of the bacteria, and
c) isolation of the L-amino acid.

10. Process according to claim 9, **characterized in that** bacteria in which further genes of the biosynthesis pathway of the desired L-amino acid are additionally enhanced are employed.

11. Process according to claim 9, **characterized in that** bacteria in which the metabolic pathways which reduce the formation of the desired L-amino acid are at least partly eliminated are employed.

12. Process according to claim 9, **characterized in that** a strain transformed with a plasmid vector is employed, and the plasmid vector carries the nucleotide sequence which codes for the ilvE gene.

13. Process according to claim 9, **characterized in that** the expression of the polynucleotide(s) which code(s) for the ilvE gene is enhanced, in particular over-expressed.

14. Process according to claim 9, **characterized in that** the regulatory or catalytic properties of the polypeptide (enzyme protein) for which the polynucleotide ilvE codes are increased.

15. Process according to claim 9, **characterized in that** for the preparation of L-amino acids, coryneform microorganisms in which at the same time one or more of the endogenous genes chosen from the group consisting of
15.1 the dapA gene which codes for dihydrodipicolinate synthase,
15.2 the gap gene which codes for glyceraldehyde 3-phosphate dehydrogenase,
15.3 the tpi gene which codes for triose phosphate isomerase,
15.4 the pgk gene which codes for 3-phosphoglycerate kinase,
15.5 the zwf gene which codes for glucose 6-phosphate dehydrogenase,
15.6 the pyc gene which codes for pyruvate carboxylase,
15.7 the mqo gene which codes for malate-quinone oxidoreductase,
15.8 the lysC gene which codes for a feed-back resistant aspartate kinase,
15.9 the lysE gene which codes for lysine export,
15.10 the hom gene which codes for homoserine dehydrogenase
15.11 the ilvA gene which codes for threonine dehydratase or the ilvA(Fbr) allele which codes for a feed back resistant threonine dehydratase,
15.12 the ilvBN gene which codes for acetohydroxy-acid synthase,
15.13 the ilvD gene which codes for dihydroxy-acid dehydratase,
15.14 the zwa1 gene which codes for the Zwa1 protein
is or are enhanced or over-expressed are fermented.

16. Process according to claim 9, **characterized in that** for the preparation of L-amino acids, coryneform microorganisms in which at the same time one or more of the genes chosen from the group consisting of
16.1 the pck gene which codes for phosphoenol pyruvate carboxykinase,
16.2 the pgi gene which codes for glucose 6-phosphate isomerase,
16.3 the poxB gene which codes for pyruvate oxidase
16.4 the zwa2 gene which codes for the Zwa2 protein is or are attenuated are fermented.

17. Coryneform bacteria which contain a vector which carries a polynucleotide according to claim 1.

18. Process according to one or more of claims 9-16, **characterized in that** microorganisms of the species Corynebacterium glutamicum are employed.

19. Process for discovering RNA, cDNA and DNA in order to isolate nucleic acids or polynucleotides or genes which code for transaminase E or have a high similarity with the sequence of the ilvE gene, **characterized in that** the polynucleotide comprising the polynucleotide sequences according to claims 1, 2, 3 or 4 is employed as hybridization probes.

20. Process according to claim 19, **characterized in that** arrays, micro arrays or DNA chips are employed.


SEQUENCE PROTOCOL

<110> Degussa AG

<120> Nucleotide sequences which code for the ilvE gene

<130> 000759 BT

<140>
<141>

<160> 2

<170> PatentIn Ver. 2.1

<210> 1
<211> 1453
<212> DNA
<213> Corynebacterium glutamicum

<220>
<221> CDS
<222> (221)..(1321)
<223> ilvE gene

<400> 1
ccttggttgg tgctgttgtg ctgtaggcat ttttcgccat tgaaagctga gtcctctcgt 60

tgaagttgtg tctccgcttt ggttggggga ggcatcaaat tgaaactaac ttttaacaag 120

cctagccatt cctcaaaacc gtgagacgaa attggctatt catcccataa aatggggctg 180

actagtgtat ctgtcaggta gcaggtgtac cttaaaatcc atg acg tca tta gag 235
Met Thr Ser Leu Glu
1               5

ttc aca gta acc cgt acc gaa aat ccg acg tca ccc gat cgt ctg aag 283
Phe Thr Val Thr Arg Thr Glu Asn Pro Thr Ser Pro Asp Arg Leu Lys
                10                  15                  20

gaa att ctt gcc gca ccg aag ttc ggt aag ttc ttc acc gac cac atg 331
Glu Ile Leu Ala Ala Pro Lys Phe Gly Lys Phe Phe Thr Asp His Met
            25                  30                  35

gtg acc att gac tgg aac gag tcg gaa ggc tgg cac aac gcc caa tta 379
Val Thr Ile Asp Trp Asn Glu Ser Glu Gly Trp His Asn Ala Gln Leu
        40                  45                  50

gtg cca tac gcg ccg att cct atg gat cct gcc acc acc gta ttc cac 427
Val Pro Tyr Ala Pro Ile Pro Met Asp Pro Ala Thr Thr Val Phe His
    55                  60                  65

tac gga cag gca att ttt gag gga att aag gcc tac cgc cat tcg gac 475
Tyr Gly Gln Ala Ile Phe Glu Gly Ile Lys Ala Tyr Arg His Ser Asp
70                  75                  80                  85

gaa acc atc aag act ttc cgt cct gat gaa aac gcc gag cgt atg cag 523
Glu Thr Ile Lys Thr Phe Arg Pro Asp Glu Asn Ala Glu Arg Met Gln
                90                  95                  100

cgt tca gca gct cga atg gca atg cca cag ttg cca acc gag gac ttt 571
Arg Ser Ala Ala Arg Met Ala Met Pro Gln Leu Pro Thr Glu Asp Phe
            105                 110                 115

att aaa gca ctt gaa ctg ctg gta gac gcg gat cag gat tgg gtt cct 619
Ile Lys Ala Leu Glu Leu Leu Val Asp Ala Asp Gln Asp Trp Val Pro
        120                 125                 130

gag tac ggc gga gaa gct tcc ctc tac ctg cgc cca ttc atg atc tcc 667
Glu Tyr Gly Gly Glu Ala Ser Leu Tyr Leu Arg Pro Phe Met Ile Ser
    135                 140                 145

acc gaa att ggc ttg ggt gtc agc cca gct gat gcc tac aag ttc ctg 715
Thr Glu Ile Gly Leu Gly Val Ser Pro Ala Asp Ala Tyr Lys Phe Leu
150                 155                 160                 165

gtc atc gca tcc cca gtc ggc gct tac ttc acc ggt gga atc aag cct 763
Val Ile Ala Ser Pro Val Gly Ala Tyr Phe Thr Gly Gly Ile Lys Pro
                170                 175                 180

gtt tcc gtc tgg ctg agc gaa gat tac gtc cgc gct gca ccc ggc gga 811
Val Ser Val Trp Leu Ser Glu Asp Tyr Val Arg Ala Ala Pro Gly Gly
            185                 190                 195

act ggt gac gcc aaa ttt gct ggc aac tac gcg gct tct ttg ctt gcc 859
Thr Gly Asp Ala Lys Phe Ala Gly Asn Tyr Ala Ala Ser Leu Leu Ala
        200                 205                 210

cag tcc cag gct gcg gaa aag ggc tgt gac cag gtc gta tgg ttg gat 907
Gln Ser Gln Ala Ala Glu Lys Gly Cys Asp Gln Val Val Trp Leu Asp
    215                 220                 225

gcc atc gag cac aag tac atc gaa gaa atg ggt ggc atg aac ctt ggg 955
Ala Ile Glu His Lys Tyr Ile Glu Glu Met Gly Gly Met Asn Leu Gly
230                 235                 240                 245

ttc atc tac cgc aac ggc gac caa gtc aag cta gtc acc cct gaa ctt 1003
Phe Ile Tyr Arg Asn Gly Asp Gln Val Lys Leu Val Thr Pro Glu Leu
                250                 255                 260

tcc ggc tca cta ctt cca ggc atc acc cgc aag tca ctt cta caa gta 1051
Ser Gly Ser Leu Leu Pro Gly Ile Thr Arg Lys Ser Leu Leu Gln Val
            265                 270                 275

gca cgc gac ttg gga tac gaa gta gaa gag cga aag atc acc acc acc 1099
Ala Arg Asp Leu Gly Tyr Glu Val Glu Glu Arg Lys Ile Thr Thr Thr
        280                 285                 290

gag tgg gaa gaa gac gca aag tct ggc gcc atg acc gag gca ttt gct 1147
Glu Trp Glu Glu Asp Ala Lys Ser Gly Ala Met Thr Glu Ala Phe Ala
    295                 300                 305

tgc ggt act gca gct gtt atc acc cct gtt ggc acc gtg aaa tca gct 1195
Cys Gly Thr Ala Ala Val Ile Thr Pro Val Gly Thr Val Lys Ser Ala
310                 315                 320                 325

cac ggc acc ttc gaa gtg aac aac aat gaa gtc gga gaa atc acg atg 1243
His Gly Thr Phe Glu Val Asn Asn Asn Glu Val Gly Glu Ile Thr Met
                330                 335                 340

aag ctt cgt gaa acc ctc acc gga att cag caa gga aac gtt gaa gac    1291
Lys Leu Arg Glu Thr Leu Thr Gly Ile Gln Gln Gly Asn Val Glu Asp
            345                 350                 355

caa aac gga tgg ctt tac cca ctg gtt ggc taaatcaacc ggttttaaga    1341
Gln Asn Gly Trp Leu Tyr Pro Leu Val Gly
        360                 365

ccccgctgca ttaaaccctg atttattgca gcggggtttt tgcgttgaca agctcttatg 1401

agacgtaggg ggtggaagca ggggtaggac gtgtccagcc caagtggcat gc        1453

<210> 2
<211> 367
<212> PRT
<213> Corynebacterium glutamicum

<400> 2
Met Thr Ser Leu Glu Phe Thr Val Thr Arg Thr Glu Asn Pro Thr Ser
1               5                   10                  15

Pro Asp Arg Leu Lys Glu Ile Leu Ala Ala Pro Lys Phe Gly Lys Phe
            20                  25                  30

Phe Thr Asp His Met Val Thr Ile Asp Trp Asn Glu Ser Glu Gly Trp
        35                  40                  45

His Asn Ala Gln Leu Val Pro Tyr Ala Pro Ile Pro Met Asp Pro Ala
    50                  55                  60

Thr Thr Val Phe His Tyr Gly Gln Ala Ile Phe Glu Gly Ile Lys Ala
65                  70                  75                  80

Tyr Arg His Ser Asp Glu Thr Ile Lys Thr Phe Arg Pro Asp Glu Asn
                85                  90                  95

Ala Glu Arg Met Gln Arg Ser Ala Ala Arg Met Ala Met Pro Gln Leu
            100                 105                 110

Pro Thr Glu Asp Phe Ile Lys Ala Leu Glu Leu Leu Val Asp Ala Asp
        115                 120                 125

Gln Asp Trp Val Pro Glu Tyr Gly Gly Glu Ala Ser Leu Tyr Leu Arg
    130                 135                 140

Pro Phe Met Ile Ser Thr Glu Ile Gly Leu Gly Val Ser Pro Ala Asp
145                 150                 155                 160

Ala Tyr Lys Phe Leu Val Ile Ala Ser Pro Val Gly Ala Tyr Phe Thr
                165                 170                 175

Gly Gly Ile Lys Pro Val Ser Val Trp Leu Ser Glu Asp Tyr Val Arg
            180                 185                 190

Ala Ala Pro Gly Gly Thr Gly Asp Ala Lys Phe Ala Gly Asn Tyr Ala
        195                 200                 205

Ala Ser Leu Leu Ala Gln Ser Gln Ala Ala Glu Lys Gly Cys Asp Gln
    210                 215                 220

Val Val Trp Leu Asp Ala Ile Glu His Lys Tyr Ile Glu Glu Met Gly
225                 230                 235                 240

Gly Met Asn Leu Gly Phe Ile Tyr Arg Asn Gly Asp Gln Val Lys Leu
                245                 250                 255

Val Thr Pro Glu Leu Ser Gly Ser Leu Leu Pro Gly Ile Thr Arg Lys
            260                 265                 270

Ser Leu Leu Gln Val Ala Arg Asp Leu Gly Tyr Glu Val Glu Glu Arg
        275                 280                 285

Lys Ile Thr Thr Thr Glu Trp Glu Glu Asp Ala Lys Ser Gly Ala Met
    290                 295                 300

Thr Glu Ala Phe Ala Cys Gly Thr Ala Ala Val Ile Thr Pro Val Gly
305                 310                 315                 320

Thr Val Lys Ser Ala His Gly Thr Phe Glu Val Asn Asn Asn Glu Val
                325                 330                 335

Gly Glu Ile Thr Met Lys Leu Arg Glu Thr Leu Thr Gly Ile Gln Gln
            340                 345                 350

Gly Asn Val Glu Asp Gln Asn Gly Trp Leu Tyr Pro Leu Val Gly
        355                 360                 365